# EUROPEAN PATENT SPECIFICATION

(11) **EP 0 619 178 B1**
(45) Date of publication and mention of the grant of the patent: **12.04.2000**
(21) Application number: 93922654.4
(22) Date of filing: 19.10.1993
(51) Int. Cl.: B32B 9/00, B65D 65/40

(54) **PROCESS FOR PRODUCING MULTILAYERED FILM MATERIALS**
VERFAHREN ZUR HERSTELLUNG MEHRSCHICHTIGER FILMMATERIALIEN
PROCEDE DE FABRICATION DE FILMS MULTICOUCHES

(30) Priority: 20.10.1992 JP 28157092
(43) Date of publication of application: 12.10.1994
(73) Proprietor: TOPPAN PRINTING CO., LTD., Tokyo 110 (JP); OTSUKA PHARMACEUTICAL FACTORY, INC., Naruto-shi, Tokushima 772-8601 (JP)
(72) Inventor: HIRAYAMA, Masahiro, Taito-ku, Tokyo 110 (JP); INOUE, Fujio, Naruto-shi, Tokushima 772 (JP); IZUMI, Masamitsu, Naruto-shi, Tokushima 772 (JP); KASHIYAMA, Shigetoshi, Naruto-shi, Tokushima 772 (JP)
(74) Representative: Fiener, Josef
(86) International application number: JP9301501
(87) International publication number: WO9408782

(56) References cited:
- EP-A- 0 372 489
- JP-A- 1 267 032
- JP-A- 2 034 328
- JP-A- 4 239 461
- DATABASE WPI Week 9239, Derwent Publications Ltd., London, GB; AN 92-319526 (39) & JP-A-4 223 153 (DAINIPPON PRINTING CO LTD) 13 August 1992
- PATENT ABSTRACTS OF JAPAN vol. 013, no. 512 (M-893) 16 November 1989 & JP-A-01 206 036 (OIKE KOGYO KK) 18 August 1989
- DATABASE WPI Week 8930, Derwent Publications Ltd., London, GB; AN 89-217469 (30) & JP-A-1 156 054 (MITSUBISHI MONSANTO KK) 19 June 1989

## Description

### TECHNICAL FIELD

This invention relates to a packaging multilayered film material. More particularly, it is concerned with a multilayered film material that is transparent, has superior gas barrier properties and water-vapor barrier properties, and is suited for the packaging of articles such as medical supplies liable to be affected by oxygen or water vapor and which must be seen through packages.

### BACKGROUND ART

As filmy packaging materials having superior gas barrier properties and water-vapor barrier properties, widely used are packaging materials comprised of a polyolefin film and laminated thereto an aluminum foil having barrier properties to oxygen and water vapor, resin film materials formed of polyethylene terephthalate, polyvinylidene chloride, polyvinyl alcohol, polyamide, an ethylene/vinyl acetate copolymer saponified product or the like, or laminated film materials thereof.

The filmy packaging materials making use of aluminum foil, however, are not transparent, and hence there has been a disadvantage that their contents can not be visually seen from the outside when filmy packages are prepared using such materials. When packages are formed from such filmy packaging materials, it is substantially impossible to employ a process other than cold forming, and hence there has been another disadvantage that the restriction on the forming process imposes a limitation to the shape of packages. When filmy packaging materials having been used are thermally disposed, there is still another disadvantage that oxides of aluminum foil turn into lumps and remain as residues of thermal disposal.

As for the resin film materials having gas barrier properties and water-vapor barrier properties or the laminated film materials thereof, they are transparent, can be formed by various methods and has a high freedom of shapes, but have achieved no satisfactory gas barrier properties or water-vapor barrier properties to medical supplies which are very liable to undergo changes of properties because of oxygen or water vapor. More specifically, the medical supplies very liable to undergo changes of properties require very high gas barrier properties as specified by an oxygen transmission of not more than 0.1 cc/(m²·24hrs·atm) in an environment of 25°C and 70%RH, and very high water-vapor barrier properties as specified by a water vapor transmission of not more than 0.2 g/(m²·24hrs) in an environment of 40°C and 90%RH. It has been impossible for the above film materials to meet such requirements. For example, resin film materials comprising polyvinyl alcohol or an ethylene/vinyl acetate copolymer saponified product have very good gas barrier properties, but their physical properties tend to be adversely affected by humidity in the environment in which they stand, resulting in a great lowering of gas barrier properties and water-vapor barrier properties.

Accordingly, various attempts to impart a high degree of gas barrier properties and water-vapor barrier properties to the resin film materials or laminated film materials are made so that the packaging materials can be endowed with both the gas barrier properties and the water-vapor barrier properties while ensuring transparency. For example, proposals are made on a composite film comprised of a film base formed of an oriented polyester film or oriented polyamide film and a silicon oxide deposited on the film base (Japanese Patent Publication No. 53-12953) and a laminated composite film comprising the composite film to which another resin film has been laminated (Japanese Utility Model Publication No. 53-42310).

In these composite films, however, a silicon oxide deposited layer have had to be formed in a thickness larger than 2,000 angstroms in order to achieve the oxygen transmission of not more than 0.1 cc/(m²·24hrs·atm). This causes a decrease in flexibility of the composite films themselves and also causes the silicon oxide deposited layer to color in amber, bringing about the problem that the color tone of contents can not be correctly recognized.

To overcome this problem, it is proposed to use an oriented polyvinyl alcohol film as a film base that can achieve the oxygen transmission of not more than 0.1 cc/(m²·24hrs·atm) even if the silicon oxide deposited layer is formed in a thickness of 2,000 angstroms or less (Japanese Patent Application Laid-open No. 1-95038).

In this case, however, the water-vapor transmission can not be made lower than the stated value, and pinholes tend to occur depending on the conditions for forming the silicon oxide deposited layer, compared with the case when films of polyethylene terephthalate and so forth are used. This has caused the problem that not only the water-vapor barrier properties but also the gas barrier properties are gradually lowered.

The present invention was made to solve the problems as discussed above, involved in the prior art. An object thereof is to provide a heat-sealable multilayered film material making use of a transparent and substantially colorless silicon oxide deposited film, having superior gas barrier properties with an oxygen transmission of not more than 0.1 cc/(m²·24hrs·atm) and superior water-vapor barrier properties with a water-vapor transmission of not more than 0.2 g/(m²·24hrs), and suited for the packaging of medical supplies very liable to undergo changes of properties.

### DISCLOSURE OF THE INVENTION

The present inventors have discovered that, when an oriented polyvinyl alcohol film or ethylene/vinyl acetate copolymer saponified product film capable of achieving the oxygen transmission of not more than 0.1 cc/(m²·24hrs·atm) in an environment of 25°C and 70%RH even if the silicon oxide deposited layer is formed in a thickness of 2,000 angstroms or less is used as a film base on which the silicon oxide is deposited, the intended gas barrier properties can be imparted to the multilayered film material comprising a composite film with a structure in which the silicon oxide deposited layer is formed on the film base. They have also discovered that superior water-vapor barrier properties and mechanical properties can be imparted to the multilayered film material when a transparent resin film with good mechanical properties which serves as a protective layer forming the external side when packaged is laminated to the silicon oxide deposited layer of the composite film. They have still also discovered that the above object can be achieved when a heat-sealable polyolefin film as a layer coming into touch with contents when packaged is laminated to the composite film on its film base side. They have thus accomplished the present invention.

The present invention provides a multilayered film material comprising a protective layer, an adhesive layer, a silicon oxide deposited layer, a polyvinyl alcohol layer or ethylene/vinyl acetate copolymer saponified product layer, an adhesive layer and a heat-sealable polyolefin layer which are laminated in this order, said layers each being transparent.

The matter for which protection is sought is defined in claims 1 and 2.

### BRIEF DESCRIPTION OF THE DRAWINGS

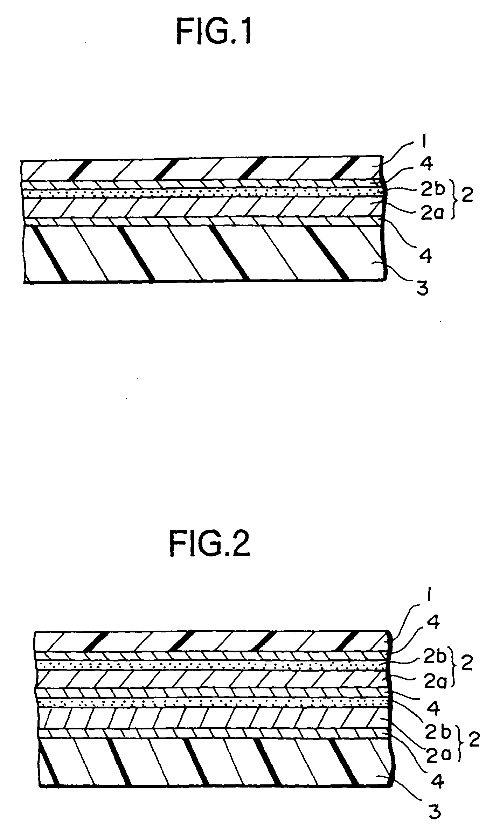

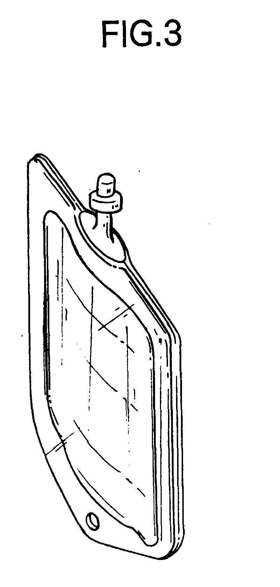

Fig. 1 is a cross-sectional view of a multilayered film material according to an embodiment of the present invention; Fig. 2 is a cross-sectional view of a multilayered film material according to another embodiment of the present invention; Fig. 3 is a perspective view of a transfusion container formed of the multilayered film material of the present invention.

### BEST MODE FOR WORKING THE INVENTION

The present invention will be described below in detail with reference to the accompanying drawings.

Fig. 1 is a cross-sectional view of a multilayered film material according to a preferred embodiment of the present invention. As shown in the drawing, the multilayered film material of the present invention comprises a protective layer 1, a composite film 2, a heat-sealable polyolefin layer 3 and adhesive layers 4 provided between them.

In the multilayered film material of the present invention, the composite film 2 is a layer capable of imparting gas barrier properties and water-vapor barrier properties to the multilayered film material, and is comprised of a film base 2a comprising a transparent polyvinyl alcohol film or ethylene/vinyl acetate copolymer saponified product film and a transparent silicon oxide deposited layer 2b formed on the film base.

As the polyvinyl alcohol film or ethylene/vinyl acetate copolymer saponified product film, it is preferable in view of gas barrier properties and mechanical strength such as dimensional stability to use a film stretched or oriented by three times or more at least in one direction. This film may preferably have a thickness of from 10 to 100 µm, and more preferably from 10 to 50 µm.

In the case when the ethylene/vinyl acetate copolymer saponified product film is used as the film base 2a, it is preferable in view of gas barrier properties to use a film having a degree of saponification of 99% or more.

The silicon oxide deposited layer 2b is a layer mainly composed of silicon monoxide, having a layer thickness of from 400 to 2,000 angstroms, and preferably from 450 to 1,000 angstroms. If the layer thickness is smaller than 400 angstroms, the layer may have insufficient gas barrier properties, and if it is larger than 2,000 angstroms, the composite film 2 may cause curling or conspicuous coloring.

Such a silicon oxide deposited layer 2b can be formed by a dry plating process such as vacuum deposition or sputtering, using silicon monoxide as a starting material. For example, in the case of vacuum deposition commonly available, the layer can be formed by heating silicon monoxide by an electron beam irradiation method or a high-frequency induction heating resistance method at a degree of vacuum of from 10⁻³ to 10⁻⁵ Torr.

The silicon oxide deposited layer 2b of the composite film 2 is provided on the side of the protective layer 1. If it is provided on the side of the heat-sealable polyolefin layer 3, the adhesive layer 4 may react with water in the film base 2a to cause bubbles.

In the present invention, the protective layer 1 is a layer that forms the external side of a receptacle such as a package produced from the multilayered film material, and is capable of protecting the composite film 2 from moisture in the atmosphere and from external impact. Such a protective layer 1 may preferably be formed using a resin film that is transparent and glossy and also has good mechanical properties (e.g., tensile strength). As the resin film, biaxially oriented polyethylene terephthalate films, polyamide films or polypropylene films may preferably be used. Of these, polyethylene terephthalate films with ultraviolet light screening properties are particularly preferred for the purpose of preventing contents from deterioration due to ultraviolet light. Such a resin film may usually have a thickness of from 10 to 30 µm, and preferably from 10 to 20 µm.

On the protective layer 1, a thin layer of polyvinylidene chloride or the like may also be formed. A printed layer may also be formed on the surface of its composite film 2 side.

In the present invention, the heat-sealable polyolefin layer 3 is a layer that forms the internal side of a package produced from the multilayered film material and comes in touch with contents of the package, and also a layer capable of imparting heat sealability to the multilayered film material. As the heat-sealable polyolefin layer 3, linear low-density polyethylene films, low-density polyethylene films, ethylene copolymer films and polypropylene films may preferably be used, which have excellent heat-sealability and hot tack and also may little affect the contents. Of these, linear low-density polyethylene films are particularly preferred. Such a polyolefin film may have a thickness of from 30 to 100 µm, and preferably from 40 to 70 µm, in view of sealability and flexibility.

In the present invention, the adhesive layer 4 is a layer capable of bonding the protective layer 1 to the composite film 2 and the composite film 2 to the heat-sealable polyolefin layer 3. Such an adhesive layer 4 is formed of an adhesive that is transparent and has a high adhesive strength. For example, urethane adhesives and polyester adhesives are preferred. In particular, two-pack hardening type urethane adhesives are preferred from the viewpoint of safety and transparency.

The adhesive layer 4 may preferably be formed using the adhesive in a coating weight of from 1 to 5 g/m².

In the foregoing, an embodiment in which the multilayered film material comprises one composite film 2 has been described. The multilayered film material of the present invention may also comprise two or more composite films. For example, as shown in Fig. 2, two sheets of composite films 2 may be so laminated as to be held between the protective layer 1 and the heat-sealable polyolefin layer 3 via the adhesive layers 4. When two or more composite films 2 are laminated in this way, gas barrier properties and water-vapor barrier properties can be more improved and also there can be almost no influence even if any pinholes occur in the silicon oxide deposited layer. In this instance, the respective silicon oxide deposited layers 2b of the two composite film are provided on the side of the protective layer 1.

The multilayered film material of the present invention can be produced by utilizing known means. For example, the silicon oxide deposited layer is formed on the polyvinyl alcohol film by vacuum deposition to firstly prepare the composite film, and a urethane adhesive is coated on the silicon oxide deposited layer of the composite film, followed by dry lamination of the protective layer such as polyethylene terephthalate film to the surface of the adhesive coating. Next, an urethane adhesive is coated on the composite film on its polyvinyl alcohol film side, followed by dry lamination of the heat-sealable polyolefine layer 3 such as polyethylene film.

The multilayered film material of the present invention thus obtained can be formed into known packages as exemplified by three-side sealed bags, four-side sealed bags, gazette bags and standing pouches. It can be also formed, as shown in Fig. 3, into a transfusion container for holding glucose solution, Ringer's solution and other pharmaceutical infusion solutions.

In the package produced from the multilayered film material of the present invention, articles such as medical supplies and food may be directly enclosed. The package may also be used as an outer package used to enclose the whole of a single or plurality of packages previously set up.

As described above, the multilayered film material of the present invention employs the oriented polyvinyl alcohol layer or ethylene/vinyl acetate copolymer saponified product layer as a film on which a silicon oxide is deposited, and hence it can achieve the oxygen transmission of not more than 0.1 cc/(m²·24hrs·atm), and preferably 0.05 cc/(m²·24hrs·atm), even when the silicon oxide deposited layer is formed in a thickness of 2,000 angstroms or less. In addition, as an protective layer forming the external side when packaged, the resin film having excellent mechanical strength is laminated to the surface of the silicon oxide deposited layer, and hence the film materials can have a water-vapor transmission of 0.2 g/(m²·24hrs) or less, and preferably 0.05 g/(m²·24hrs) or less.

The heat-sealable polyolefin layer is also used as an inner layer coming in touch with contents when packaged, and hence heat sealing can be carried out without substantially no ill influence on the contents.

Moreover, the respective component layers are transparent and substantially not colored, and hence the state of contents packaged therein can be visually correctly recognized.

### EXAMPLES

The present invention will be described below in greater detail by giving Examples.

### Example 1

One side of a 12 µm thick biaxially oriented polyethylene terephthalate film was subjected to corona treatment and printing was applied to the corona-treated surface to prepare a protective layer film.

Meanwhile, on a 15 µm thick biaxially oriented polyvinyl alcohol film (stretch ratio: 3 times), a 550 angstrom thick silicon oxide deposited layer was formed by vacuum deposition to produce a composite film.

On the silicon oxide deposited layer of this composite film, a two-pack hardening type urethane adhesive (a 10:1 mixture, in weight ratio, of a main agent TAKELUCK A515, available from Takeda Chemical Industries, Ltd., and a hardener TAKENATE, available from Takeda Chemical Industries, Ltd.) was coated in a coating weight of 2.5 g/m². On the surface of the resulting adhesive coating, the protective layer film was overlaid with its printed surface face-to-face, and the composite film and the protective layer film were laminated to each other by dry lamination.

On the polyvinyl alcohol film of the resulting laminate, the two-pack hardening type urethane adhesive described above was coated in a coating weight of 2.5 g/m². To the surface of the resulting adhesive coating, a 50 µm thick linear low-density polyethylene film was laminated as an heat-sealable polyolefin layer film by dry lamination. Thus, a multilayered film material with the structure as shown in Fig. 1 was produced.

With respect to the multilayered film material thus obtained, its oxygen transmission was measured in an environment of 25°C and 70%RH using an oxygen transmission measuring device (OX-TRAN 10/50A, manufactured by Modern Controls, Inc.), and also its water-vapor transmission was measured in an environment of 40°C and 90%RH using a water-vapor transmission measuring device (PERMATRAN-W TWIN, manufactured by Modern Controls, Inc.). As a result, the oxygen transmission was 0.1 cc/(m²·24hrs·atm) and the water-vapor transmission was 0.2 g/(m²·24hrs), showing superior properties.

### Example 2

One side of a 12 µm thick biaxially oriented polyethylene terephthalate film was subjected to corona treatment and printing was applied to the corona-treated surface to prepare a protective layer film.

Meanwhile, on a 15 µm thick biaxially oriented polyvinyl alcohol film (stretch ratio: 3 times), a 550 angstrom thick silicon oxide deposited layer was formed by vacuum deposition to produce a first composite film.

On the silicon oxide deposited layer of this first composite film, a two-pack hardening type urethane adhesive (a 10:1 mixture, in weight ratio, of a main agent TAKELUCK A515, available from Takeda Chemical Industries, Ltd., and a hardener TAKENATE, available from Takeda Chemical Industries, Ltd.) was coated in a coating weight of 2.5 g/m². On the surface of the resulting adhesive coating, the protective layer film was overlaid with its printed surface face-to-face, and the first composite film and the protective layer film were laminated to each other by dry lamination to produce laminate A.

Next, the same procedure for the production of the first composite film was repeated to produce a second composite film.

On the silicon oxide deposited layer of this second composite film, the same two-pack hardening type urethane adhesive as the above was coated in a coating weight of 2.5 g/m². On the surface of the resulting adhesive coating, the laminate A was overlaid with its polyvinyl alcohol film surface face-to-face, and the second composite film and the laminate A were laminated to each other by dry lamination to produce laminate B.

On the polyvinyl alcohol film of the resulting laminate B, the two-pack hardening type urethane adhesive described above was coated in a coating weight of 2.5 g/m². To the surface of the resulting adhesive coating, a 50 µm thick linear low-density polyethylene film was laminated as an heat-sealable polyolefin layer film by dry lamination. Thus, a multilayered film material with the structure as shown in Fig. 2 was produced.

With respect to the multilayered film material thus obtained, its oxygen transmission and water-vapor transmission were measured in the same manner as in Example 1. As a result, the oxygen transmission was less than 0.1 cc/(m²·24hrs·atm) which was lower than a limit of detection of the measuring device, and the water-vapor transmission was less than 0.1 g/(m²·24hrs) which was also lower than a limit of detection, showing superior properties.

### Example 3

Example 1 was repeated to produce a multilayered film material with the structure as shown in Fig. 1, except that as a protective layer film the biaxially oriented polyethylene terephthalate film was replaced with a 12 µm thick biaxially oriented polypropylene film.

With respect to the multilayered film material thus obtained, its oxygen transmission and water-vapor transmission were measured in the same manner as in Example 1. As a result, the oxygen transmission was 0.1 cc/(m²·24hrs·atm) and the water-vapor transmission was less than 0.1 g/(m²·24hrs), showing superior properties.

### Example 4

Example 1 was repeated to produce a multilayered film material with the structure as shown in Fig. 1, except that the biaxially oriented polyvinyl alcohol film of the composite film was replaced with a 15 µm thick biaxially oriented ethylene/vinyl acetate copolymer saponified product film (ethylene content: 37%; degree of saponification: 99.5%; stretch ratio: 3 times).

With respect to the multilayered film material thus obtained, its oxygen transmission and water-vapor transmission were measured in the same manner as in Example 1. As a result, the oxygen transmission was 0.1 cc/(m²·24hrs·atm) and the water-vapor transmission was 0.2 g/(m²·24hrs), showing superior properties.

### Example 5

Example 1 was repeated to produce a multilayered film material with the structure as shown in Fig. 1, except that the linear low-density polyethylene film was replaced with a 40 µm low-density polyethylene film.

With respect to the multilayered film material thus obtained, its oxygen transmission and water-vapor transmission were measured in the same manner as in Example 1. As a result, the oxygen transmission was 0.1 cc/(m²·24hrs·atm) and the water-vapor transmission was 0.2 g/(m²·24hrs), showing superior properties.

### Comparative Example 1

Example 1 was repeated to produce a multilayered film material, except that the silicon oxide deposited layer of the composite film was provided on the side of the linear low-density polyethylene film.

The multilayered film material thus obtained caused a large number of bubbles in the layers comprising the adhesive, and was unsuitable for practical use.

### Comparative Example 2

Example 2 was repeated to produce a multilayered film material, except that the silicon oxide deposited layers of the two composite films were put face-to-face.

The multilayered film material thus obtained caused a large number of bubbles in the layers comprising the adhesive, and was unsuitable for practical use.

### Comparative Example 3

Example 1 was repeated to produce a multilayered film material with the structure as shown in Fig. 1, except that the polyvinyl alcohol film as the film base of the composite film was replaced with a 12 pm thick polyethylene terephthalate film and the silicon oxide deposited layer was formed in a thickness of 1,500 angstroms.

With respect to the multilayered film material thus obtained, its oxygen transmission and water-vapor transmission were measured in the same manner as in Example 1. As a result, the oxygen transmission was 0.1 cc/(m²·24hrs·atm) but the water-vapor transmission was as very high as 0.5 g/(m²·24hrs), and the product was unsuitable for practical use.

### Comparative Example 4

Comparative Example 3 was repeated to produce a multilayered film material with the structure as shown in Fig. 2, except that two composite films were used.

With respect to the multilayered film material thus obtained, its oxygen transmission and water-vapor transmission were measured in the same manner as in Example 1. As a result, the oxygen transmission was 0.1 cc/(m²·24hrs·atm) and the water-vapor transmission was 0.09 g/(m²·24hrs), showing satisfactory values. However, the product conspicuously colored. A baglike container was produced using this material. As a result, the surface state of its contents was not visually recognizable.

### Packaging Example 1

The multilayered film material obtained in Example 1 was cut into substantially rectangular sheets. Two sheets of the multilayered film material were put together so as for their heat-sealable polyolefin layers to be face-to-face, and were heat-sealed at their circumference except a part thereof to produce a bag.

This bag was filled with 1,000,000 units (about 625 mg) of benzylpenicillin potassium (hereinafter "PC-G") and 4 g of dried silica gel in an atmosphere of nitrogen, and sealed using an impulse sealer. Package A was thus obtained.

This package A was put in a thermo-hygrostat (PR-4FT, manufactured by Tabai Espec Corp.) set at 60°C and 75%RH and stored therein. Then the potency, color and moisture content (%) of PC-G in the package were measured right before the filling with PC-G, on lapse of a week, on lapse of two weeks and on lapse of four weeks. Results of the measurement are shown in Table 1.

The potency of PC-G (the potency at the filling is regarded as 100) was measured by the cylinder-plate method, and the color and moisture content thereof were measured according to what is prescribed in "RAPJ" (Requirements for Antibiotic Products of Japan). Numerical values shown in Table 1 are average values on three samples.

For reference, package B conventionally used for the packaging of medical supplies and package C comprised of a glass vial were produced in the manner as described below, and were tested in the same manner as the package A. The package C is a package conventionally used as a container showing very good gas barrier properties and water-vapor barrier properties.

The package B was produced by repeating the same procedure as that for the production of the package A except that the multilayered film material of Example 1 was replaced with a laminated film (12 µm thick polyethylene terephthalate film coated with polyvinylidene chloride/25 µm thick polyvinylidene chloride film/45 µm thick linear low-density polyethylene film) having an oxygen transmission of 3.00 cc/(m²·24hrs·atm).

The package C was obtained in the following way: A glass vial according to "RAPJ" was filled with 1,000,000 units (about 625 mg) of PC-G in an atmosphere of nitrogen, and stoppered with a rubber stopper coated with Teflon (trademark), around which a cap made of aluminum was further rolled.

**Table 1**

| | | | Time lapsed | | |
|---|---|---|---|---|---|
| Test item | Package | Right before packaging | 1 week | 2 weeks | 4 weeks |
| Potency of PC-G: | A | 100 | 99.9 | 98.2 | 96.1 |
| | B | 100 | 74.8 | 57.3 | 25.6 |
| | C | 100 | 99.6 | 98.7 | 95.2 |
| Color of PC-G: | A | White | White | White | White |
| | B | White | Pale yellow | Yellow | Yellowish brown |
| | C | White | White | White | White |
| Moisture content: (%) | A | 0.12 | 0.14 | 0.16 | 0.15 |
| | B | 0.12 | 1.40 | 1.71 | 2.23 |
| | C | 0.12 | 0.15 | 0.13 | 0.14 |

As shown in Table 1, the package A produced from the multilayered film material of the present invention exhibited a performance comparable to the package C comprised of the glass vial used as a package having very high gas barrier properties and water-vapor barrier properties.

### Packaging Example 2

The procedure for producing the package A was repeated to obtain package D, except that the multilayered film material obtained in Example 1 was replaced with the multilayered film material obtained in Example 2 and the PC-G was replaced with cephazolin sodium cake (hereinafter "CEZ"). The procedures for producing the packages B and C were also repeated to produce packages E and F, respectively, except that the PC-G was replaced with CEZ.

These packages D, E and F were each put in a thermohygrostat (PR-4FT, manufactured by Tabsi Espec Corp.) set at 60°C and 75%RH and stored therein. Then the potency, color and moisture content (%) of CEZ in the package were measured right before the filling with CEZ, on lapse of a week, on lapse of two weeks and on lapse of four weeks. Results of the measurement are shown in Table 2.

The potency of CEZ was measured by HLPC, and the color, the moisture content and the pH thereof were measured according to what is prescribed in "RAPJ". Numerical values shown in Table 2 are average values on three samples.

**Table 2**

| | | | Time lapsed | | |
|---|---|---|---|---|---|
| Test item | Package | Right before packaging | 1 week | 2 weeks | 4 weeks |
| Potency of CEZ: | D | 930 | 924.1 | 922.7 | 923.4 |
| | E | 930 | 511.8 | 296.3 | 100.1 |
| | F | 930 | 925.2 | 924.1 | 923.8 |
| Color of CEZ: | D | White | White | White | White |
| | E | White | Yellow | Yellow | Yellow |
| | F | White | White | White | White |
| Moisture content: (%) | D | 0.68 | 0.64 | 0.57 | 0.67 |
| | E | 0.76 | 4.76 | 5.12 | 6.42 |
| | F | 0.75 | 0.58 | 0.66 | 0.61 |
| pH value: | D | 4.86 | 4.91 | 4.88 | 4.87 |
| | E | 4.79 | 4.01 | 4.20 | 4.74 |
| | F | 4.99 | 5.03 | 4.98 | 5.04 |

As shown in Table 2, the package D produced from the multilayered film material of the present invention exhibited a performance comparable to the package F comprised of the glass vial used as a package having very high gas barrier properties and water-vapor barrier properties.

### Packaging Example 3

The procedure for producing the package A was repeated to obtain package G, except that the multilayered film material obtained in Example 1 was replaced with the multilayered film material obtained in Example 2 and the PC-G was replaced with 10 g of powdered glutamine. The package G was put in a thermo-hygrostat (PR-4FT, manufactured by Tabai Espec Corp.) set at 60°C and 75%RH and stored therein for 4 weeks. After the storage, the contents were analyzed using an amino acid automatic analyzer to ascertain that no change in the content of glutamine occurred and hence the presence of glutamic acid, an oxidized product, was undetected.

### Packaging Example 4

A bag formed of 100 µm thick linear low-density polyethylene film was filled with 10 g of powdered glutamine in an atmosphere of nitrogen and then sealed to produce a package.

Meanwhile, another package was produced in the same manner as the package A except that the multilayered film material obtained in Example 1 was replaced with the multilayered film material obtained in Example 2. In the package thus produced, a smaller package comprising the package formed of linear low-density polyethylene film and in which 10 g of powdered glutamine was enclosed was put and the outer package was sealed in an atmosphere of nitrogen to obtain a double package, package H. This package H was put in a thermo-hygrostat (PR-4FT, manufactured by Tabai Espec Corp.) set at 60°C and 75%RH and stored therein for 4 weeks. After the storage, the contents were analyzed using an amino acid automatic analyzer to ascertain that no change in the content of glutamine occurred and hence the presence of glutamic acid, an oxidized product, was undetected.

### INDUSTRIAL UTILITY

The multilayered film material of the present invention has superior gas barrier properties and water-vapor barrier properties. Moreover, it is transparent and substantially not colored, and has a good heat-sealability. Thus, the present material is particularly useful for the packaging of articles very liable to undergo changes of properties because of oxygen or water vapor.

## Claims

1. A process of producing a multilayered film material for a package, comprising the steps of:
(a) providing a composite film (2) comprised of a base film (2a) and a 40-200nm (400-2000 angstrom) thick silicon oxide deposited layer (2b) formed thereon, the base film (2a) comprised of a polyvinyl alcohol film or an ethylene/vinyl acetate copolymer saponified product film;
(b) coating an adhesive (4) on the silicon oxide deposited layer (2b) of the composite film (2);
(c) laminating a protective layer (1), forming the external side of the package, on the surface of the adhesive coated silicon oxide layer (2b), by dry lamination;
(d) coating an adhesive (4) on the base film (2a) of the composite film (2); and
(e) laminating a heat-sealable polyolefin layer (3), forming the internal side of the package, on the surface of the adhesive coated base film (2a), by dry lamination.

2. A process of producing a multilayered film material for a package, comprising the steps of:
(a) providing first and second composite films (2), each of which is comprised of a base film (2a) and a 40-200nm (400-2000 angstrom) thick silicon oxide deposited layer (2b) formed thereon, the base film (2a) comprised of a polyvinyl alcohol film or an ethylene/vinyl acetate copolymer saponified product film;
(b) coating an adhesive (4) on the silicon oxide deposited layer (2b) of the first composite film (2);
(c) laminating a protective layer (1) on the surface of the adhesive coated silicon oxide layer (2b) of the first composite film (2), by dry lamination;
(d) coating an adhesive (4) on the silicon oxide deposited layer (2b) of the second composite film (2);
(e) laminating the second composite film (2) on the surface of the first composite film (2) so that the base film (2a) of the first composite film (2) comes in contact with the surface of the adhesive (4) coated second composite film (2), by dry lamination;
(f) coating an adhesive (4) on the base film (2a) of the second composite film (2);
(g) laminating a heat-sealable polyolefin layer (3) on the surface of the adhesive coated base film (2a) of the second composite film (2), by dry lamination.

## Patentansprüche

1. Verfahren zur Herstellung eines mehrschichtigen Filmmaterials für eine Verpackung, umfassend die Schritte:
(a) Bereitstellen eines Verbundfilms (2), bestehend aus einem Basisfilm (2a) und einer darauf ausgebildeten 40 - 200 mm (400 - 2000 Å) dicken Siliciumoxid-Ablagerungsschicht (2b), wobei der Basisfilm (2a) aus einem Polyvinylalkoholfilm oder einem Film aus dem Verseifungsprodukt von Etyhlen/Vinylacetatcopolymer besteht;
(b) Beschichten eines Haftträgers (4) auf die Siliciumoxid-Ablagerungsschicht (2b) des Verbundfilms (2);
(c) Laminieren einer die Außenseite der Verpackung bildenden Schutzschicht (1) durch Trockenlaminierung auf die Oberfläche der haftträgerbeschichteten Siliciumoxidschicht (2b);
(d) Beschichten eines Haftträgers (4) auf den Basisfilm (2a) des Verbundfilms (2); und
(e) Laminieren einer die Innenseite der Verpackung bildenden hitzeversiegelbaren Polyolefinschicht (3) durch Trockenlaminierung auf die Oberfläche des haftträgerbeschichteten Basisfilms (2a).

2. Verfahren zur Herstellung eines mehrschichtigen Filmmaterials für eine Verpackung, umfassend die Schritte:
(a) Bereitstellen eines ersten und eines zweiten Verbundfilms (2), die jeweils aus einem Basisfilm (2a) und einer darauf ausgebildeten 40 - 200 mm (400 - 2000 Å) dicken Siliciumoxid-Ablagerungsschicht (2b) bestehen, wobei der Basisfilm (2a) aus einem Polyvinylalkoholfilm oder einem Film aus dem Verseifungsprodukt von Etyhlen/Vinylacetatcopolymer besteht;
(b) Beschichten eines Haftträgers (4) auf die Siliciumoxid-Ablagerungsschicht (2b) des ersten Verbundfilms (2);
(c) Laminieren einer Schutzschicht (1) durch Trockenlaminierung auf die Oberfläche der haftträgerbeschichteten Siliciumoxidschicht (2b) des ersten Verbundfilms (2);
(d) Beschichten eines Haftträgers (4) auf die Siliciumoxid-Ablagerungsschicht (2b) des zweiten Verbundfilms (2);
(e) Laminieren des zweiten Verbundfilms (2) durch Trockenlaminierung auf die Oberfläche des ersten Verbundfilms (2), so daß der Basisfilm (2a) des ersten Verbundfilms (2) mit der Oberfläche des mit Haftträger (4) beschichteten zweiten Verbundfilms (2) in Kontakt kommt;
(f) Beschichten eines Haftträgers (4) auf den Basisfilm (2a) des zweiten Verbundfilms (2);
(g) Laminieren einer hitzeversiegelbaren Polyolefinschicht (3) durch Trockenlaminierung auf die Oberfläche des haftträgerbeschichteten Basisfilms (2a) des zweiten Verbundfilms (2).

## Revendications

1. Procédé pour la production d'un matériau de film multicouche pour un emballage comprenant les étapes consistant :
(a) à fournir un film composite (2) constitué d'un film de base (2a) et d'une couche de dépôt d'oxyde de silicium d'épaisseur 40-200 nm (400-2 000 angströms) (2b) formée sur son dessus, le film de base (2a) étant constitué d'un film de poly(alcool vinylique) ou d'un film de produit saponifié de copolymère d'éthylène/acétate de vinyle;
(b) à déposer un adhésif (4) sur la couche de dépôt d'oxyde de silicium (2b) du film composite (2);
(c) à stratifier une couche protectrice (1), formant le côté externe de l'emballage, sur la surface de la couche de dépôt d'oxyde de silicium adhésive (2b) par laminage à sec;
(d) à déposer un adhésif(4) sur le film de base (2a) du film composite (2); et
(e) à stratifier une couche de polyoléfine thermocollable (3), formant le côté interne de l'emballage, sur la surface du film de base revêtu d'adhésif (2a) par laminage à sec.

2. Procédé pour la production d'un matériau de film multicouche pour un emballage comprenant les étapes consistant :
(a) à fournir des premier et second films composites (2), chacun constitué d'un film de base (2a) et d'une couche de dépôt d'oxyde de silicium d'épaisseur 40-200 nm (400-2 000 angströms) formée sur son dessus, le film de base (2a) étant constitué d'un film de poly(alcool vinylique) ou d'un film de produit saponifié de copolymère d'éthylène/acétate de vinyle;
(b) à déposer un adhésif (4) sur la couche de dépôt d'oxyde de silicium (2b) du premier film composite (2);
(c) à stratifier une couche protectrice (1) sur la surface de la couche de dépôt d'oxyde de silicium adhésive (2b) du premier film composite (2) par laminage à sec;
(d) à déposer un adhésif (4) sur la couche de dépôt d'oxyde de silicium adhésive (2b) du second film composite (2);
(e) à stratifier le second film composite (2) sur la surface du premier film composite (2) de telle sorte que le film de base (2a) du premier film composite (2) est en contact avec la surface du second film composite (2) revêtu d'adhésif (4) par laminage à sec;
(f) à déposer un adhésif (4) sur le film de base (2a) du second film composite (2);
(g) à stratifier une couche de polyoléfine thermocollable (3) sur la surface du film de base revêtu d'adhésif (2a) du second film composite (2) par laminage à sec.
